# EUROPEAN PATENT SPECIFICATION

(11) **EP 3 489 689 B1**
(45) Date of publication and mention of the grant of the patent: **17.06.2020**
(21) Application number: 18195687.1
(22) Date of filing: 20.12.2005
(51) Int. Cl.: G01N 33/68, G01N 33/92

(54) **DETERMINATION OF NEUTROPHIL GELATINASE-ASSOCIATED LIPOCALIN (NGAL) AS A DIAGNOSTIC MARKER FOR RENAL DISORDERS**
BESTIMMUNG VON NEUTROPHILEM GELATINASE-ASSOZIIERTEM LIPOCALIN (NGAL) ALS DIAGNOSTISCHER MARKER FÜR NIERENERKRANKUNGEN
DÉTERMINATION DE LA LIPOCALINE NEUTROPHILE ASSOCIÉE À LA GÉLATINASE (NGAL) UTILE COMME MARQUEUR DIAGNOSTIC DANS LES TROUBLES RÉNAUX

(30) Priority: 20.12.2004 US 637503 P; 21.09.2005 US 719307 P
(43) Date of publication of application: 29.05.2019
(62) Divisional of application: 17152153.7
(73) Proprietor: Antibodyshop A/S, 2820 Gentofte (DK)
(72) Inventor: Uttenthal, Lars Otto, 37002 Salamanca (ES); Juanes, Margarita Ghiglione, 37002 Salamanca (ES); Bangert, Kristian, 2840 Holte (DK)
(74) Representative: Høiberg P/S

(56) References cited:
- WO-A2-2004/088276
- WO-A2-2005/121788
- JAYA MISHRA ET AL: "Neutrophil gelatinase-associated lipocalin (NGAL) as a biomarker for acute renal injury after cardiac surgery", LANCET, vol. 365, no. 9466, 1 April 2005 (2005-04-01), pages 1231-1238, XP055532326, AMSTERDAM, NL ISSN: 0140-6736, DOI: 10.1016/S0140-6736(05)74811-X
- MISHRA J ET AL: "Identification of neutrophil gelatinase-associated lipocalin as a novel early urinary biomarker for ischemic renal injury", JOURNAL OF THE AMERICAN SOCIETY OF NEPHROLOGY, WILLIAMS AND WILKINS, BALTIMORE, MD, US, vol. 14, no. 10, 1 October 2003 (2003-10-01), pages 2534-2543, XP002377943, ISSN: 1046-6673, DOI: 10.1097/01.ASN.0000088027.54400.C6
- MISHRA J ET AL: "NEUTROPHIL GELATINASE-ASSOCIATED LIPOCALIN: A NOVEL EARLY URINARY BIOMARKER FOR CISPLATIN NEPHROTOXICITY", AMERICAN JOURNAL OF NEPHROL, S. KARGER AG, BASEL, CH, vol. 24, 12 May 2004 (2004-05-12), pages 307-315, XP008063174, ISSN: 0250-4095, DOI: 10.1159/000078452
- XU S ET AL: "Lipocalins as biochemical markers of disease", BIOCHIMICA ET BIOPHYSICA ACTA, ELSEVIER, NL, vol. 1482, no. 1-2, 18 October 2000 (2000-10-18), pages 298-307, XP002376345, ISSN: 0006-3002
- L. O. Uttenthal: "NGAL: a marker molecule for the distressed kidney?", CLI, November 2005, 1 November 2005 (2005-11-01), pages 1-2, XP055532339, Retrieved from the Internet: URL:https://www.clinlabint.com/fileadmin/p df/pdf_general/ngal-a-marker-molecule-for- the-distressed-kidney.pdf [retrieved on 2018-12-10]
- MORI K ET AL: "Endocytic Delivery of Lipocalin-Siderophore-Iron Complex Rescues the Kidney from Ischemia-Reperfusion Injury", JOURNAL OF CLINICAL INVESTIGATION, B M J GROUP, GB, vol. 115, no. 3, 1 January 2005 (2005-01-01), pages 610-621, XP003013565, ISSN: 0021-9738, DOI: 10.1172/JCI200523056

## Description

This application is a non-provisional of U.S. provisional applications Serial No. 60/637,503, filed 20 December, 2004, and 60/719,307, filed 21 September 2005.

### Field of the invention

The present invention relates to methods of discriminating between a condition that does not affect the kidney, wherein the condition that does not affect the kidney is an inflammatory disorder, an infective disorder or a cancerous disorder, and the likelihood of a renal disorder, wherein the renal disorder is selected from acute renal failure, acute tubular necrosis and acute tubule-interstitial nephropathy, in a human being, said method comprising the steps of: i) determining the concentration of human neutrophil gelatinase-associatedlipocalin (NGAL) in a sample of bodily fluid from the human being, ii) comparing said concentration with a predetermined cutoff value, said cutoff value being chosen to exclude lower concentrations of NGAL associated with conditions that do not affect the kidney, wherein a concentration above the cutoff value is indicative of the likelihood of the renal disorder.

### Background of the Invention

The detection of renal ischemic injury that may result in ARF, ATN or ATIN has hitherto depended on clinical signs, such as oliguria and fluid retention, combined with the results of chemical tests on blood and urine showing a low sodium excretion, raised and rising levels of creatinine and urea in the blood and, if measured, a low creatinine clearance. These signs are indicative of the presence of the established condition. Various urinary markers of renal tubular damage have also been analyzed. Of these, alpha 1-microglobulin, beta 2-microglobulin (Penders and Delanghe JR, 2004) and N-acetyl-beta-D-glucosaminidase (NAG) (Kotanko et al., 2000) are elevated in cases of established ATN, but may only appear 4 or 5 days after the ischemic or toxic insult in experimental animals. Kidney injury molecule-1 (KIM-1) is also elevated in the urine of patients with ATN, but the time course, though probably early, has not been established in human patients (Han et al., 2002). Cysteine rich protein 61 (CYR61) peaks in the urine at 6 to 9 hours after ischemic injury in experimental animals (Muramatsu et al., 2002). However, levels in humans are still unknown. Other markers that have been studied include clusterin (Aulitzky et al., 1992) and lipocalin-type prostaglandin D synthase (L-PGDS) (Tsuchida et al., 2004); their usefulness for the early diagnosis or prediction of ATN is still unclear. As a result of the generally slow time course of appearance of these marker molecules in urine after exposure of the kidney to ischemia or nephrotoxic agents, they have not entered into general use to diagnose early or impending renal disorders resulting from these insults.

Neutrophil gelatinase-associated lipocalin (NGAL) is also known as neutrophil lipocalin (NL; HNL in the case of human neutrophil lipocalin), lipocalin 2 (LCN2), 25-kDa alpha 2-microglobulin-related protein (in the rat) or 24P3 (in the mouse). In the rat, it has also been referred to as neu-related lipocalin (NRL), as its gene is overexpressed in mammary tumors initiated by the neu (HER2/c-erbB-2) oncogene (Stoesz and Gould, 1995). NGAL is a 25-kDa glycoprotein first isolated from the granules of polymorphonuclear leukocytes (Triebel et al., 1992; Kjeldsen et al., 1993). It contains a disulfide bridge and forms a proportion of dimers and a smaller proportion of trimers. It is associated with 92-kDa human neutrophil type IV collagenase, also called matrix metalloproteinase 9 (MMP-9) or gelatinase B, either as a monomer forming a complex of apparent kDa 115, (Monier et al., 2000; Yan et al., 2001) or as a dimer, forming a complex of apparent kDa 125 (Yan et al., 2001). These complexes have been identified in the urine of patients with a variety of cancers, including cancers of the prostate, bladder, kidney and breast.

NGAL was initially disclosed as a marker of neutrophil activation, being released into the blood when invading microorganisms, in particular pyogenic bacteria, cause degranulation of the neutrophils and exocytosis of the granule proteins. As such, the measurement of elevated levels of NGAL in a plasma or serum sample from a human is believed to be indicative of the individual suffering from an inflammation, especially one caused by a bacterial infection (Venge, 2000; U.S. Patent 6,136,526; PCT application WO95/29404). In this respect, but in contradiction to its claimed specific derivation from neutrophils, NGAL (24P3) was identified as an acute phase protein of type 1 in the mouse, where expression was mainly located in the liver during the acute phase response (Liu and Nilsen-Hamilton, 1995).

US patent application 2004/0219603 describes the use of NGAL as a urinary biomarker for detecting the early onset of renal tubular cell injury. However, it is not described whether or how renal tubular cell injury can be discriminated from systemic inflammation or bacterial infection as the cause of the elevated NGAL level.

### Summary of the Invention

It has now surprisingly been found that NGAL levels due to renal injury are generally higher than levels of NGAL that result from inflammatory, infective or cancerous conditions that do not affect renal function. This has allowed the establishment of methods for the diagnosis and/or monitoring of a renal disorder in a patient which distinguish renal disorders from other disorders that do not affect the kidney. By renal disorder is meant any alteration of function, including the structural and ultrastructural correlates of that alteration, either of the kidney as a whole or of one or more cellular structures of which it is composed, that goes beyond the regulatory mechanisms which maintain the normal healthy state. Non-exclusive examples of such disorders include conditions associated with renal ischemia such as acute tubular necrosis (ATN) or acute tubulo-interstitial nephropathy (ATIN), and also include acute renal failure (ARF) or chronic renal failure (CRF) of whatever cause, acute and chronic glomerulonephritis of whatever cause, nephropathy due to urinary obstruction, nephropathy due to hypertension, nephropathy associated with pre-eclampsia or toxemia of pregnancy, rejection or recurrent disease of a transplanted kidney, as well as congenital and neoplastic diseases of the kidney.

Accordingly, the present disclosure provides methods for the diagnosis and/or monitoring of renal injury in humans, comprising measuring a level of human neutrophil gelatinase-associated lipocalin (NGAL) in a sample of bodily fluid, preferably urine, obtained from the patient. Elevated levels of NGAL are indicative of renal injury if they are higher than specified less elevated levels of NGAL that result from other disorders that do not necessarily affect renal function, such as inflammatory or infective conditions or cancers. Thus, in a first aspect, the disclosure relates to a method of diagnosing, monitoring or determining the likelihood of a renal disorder in a human being, wherein said method discriminates between a renal disorder and another condition that does not affect the kidney, said method comprising the steps of
i) determining the concentration of human NGAL in a sample of bodily fluid from the human being,
ii) comparing said concentration with a predetermined cutoff value, said cutoff value being chosen to exclude lower concentrations of NGAL associated with conditions that do not affect the kidney, wherein a concentration above the cutoff value is indicative of a renal disorder.

Furthermore, the present disclosure allows the discrimination between different degrees of renal disorder. Thus, the method comprises a further step of comparing said concentration with a second cutoff value, said second cutoff value being chosen to exclude lower concentrations of NGAL associated with a lesser degree of renal disorders that are unlikely to require treatment by dialysis, wherein a concentration above the cutoff value is indicative of a more severe disorder that is likely to require treatment by dialysis.

The analysis of other marker molecules for renal injury that may result in ARF, ATN or ATIN has not entered into routine clinical use, as these other markers appear in the urine too late in the course of development of the renal disorder to be useful in alerting the clinician to the development of the disorder or to guide preventive or therapeutic measures. This problem has been solved by the present invention which uses the analysis of NGAL in human samples such as urine to detect the development of renal disorders.

Animal studies suggest that this is the earliest marker of renal insult to appear in the urine, with levels in urine rising within 2 or 3 hours after the initiation of the insult. The analysis of urinary NGAL in accordance with the present invention thus offers a method of detecting renal injury that may result in ARF, ATN or ATIN, discriminating it from other disorders, and, if the rate of progression permits, instituting appropriate measures to reverse the process.

Levels of NGAL are preferably determined by an immunochemical method. Examples of such methods include, but are in no way limited to a sandwich ELISA (enzyme-linked immunosorbent assay), a lateral flow method, or a dipstick.

### Description of the drawings

**Figure 1** Receiver operating characteristics (ROC) curve for maximal urinary NGAL values with respect to the diagnosis of renal affection in 60 adult patients admitted to a hospital intensive care unit. Only patients that could be classified from other data as having or not having renal affection were included.
**Figure 2** Receiver operating characteristics (ROC) curve for maximal plasma NGAL values with respect to the diagnosis of renal affection in the same patients as in Figure 1.
**Figure 3** Receiver operating characteristics (ROC) curve for maximal urinary NGAL values with respect to the diagnosis of dialysis requirement in 32 adult patients admitted to a hospital intensive care unit and clinically diagnosed as having renal affection.
**Figure 4** Time course of urinary NGAL and plasma creatinine values in an elderly male patient operated acutely for rupture of an abdominal aortic aneurysm.
**Figure 5** Time course of urinary NGAL and plasma creatinine values in a female patient who developed sepsis which did not affect renal function. The urinary NGAL cutoff level (329 ng/mL) for the diagnosis of renal affection and the upper limit of normal for plasma creatinine in women (0.088 mmol/L) are shown.

### Detailed Description of the Invention

We have found that concentrations of human neutrophil gelatinase-associated lipocalin (NGAL) in samples of human urine are only influenced moderately by infective or inflammatory states. However, NGAL levels are markedly elevated in renal disorders attributed to ischemic injury and the elevation of NGAL is particularly high in ATN. Further, we have now found that serum or plasma levels of NGAL do not necessarily reflect infective or inflammatory states, and may be raised even when the blood neutrophil count is extremely low, as may occur in leukemias or as a consequence of treating leukemia. There is in fact a close correlation between the concentration of NGAL in human serum or plasma and the concentration of NGAL in urine when spot samples of blood and urine from randomly selected critically ill patients are analyzed, while there are very poor correlations of these levels with either the neutrophil count in peripheral blood or the concentration of C-reactive protein, an acute phase protein that is commonly used as a marker of inflammation. Thus, without being bound by a particular theory, it may be hypothesized that NGAL is produced by the kidneys in response to ischemia and other influences capable of stimulating renal NGAL expression, and is not only released in large amounts in the urine, but also into the blood, thus confounding the use of NGAL determinations in serum or plasma as a marker of inflammation or bacterial infection as set forth in U.S. Patent 6,136,526.

Accordingly, the present disclosure relates to measurement of NGAL in a sample of bodily fluid, preferably human urine from which any neutrophils have been removed, as a diagnostic marker of renal disorders, especially those due to renal ischemia or nephrotoxic agents. In the present disclosure, for the concentration of NGAL to be specifically indicative of renal disorder, it must exceed a cutoff value set to exclude those lower concentrations of NGAL that may result from infective or inflammatory states or carcinomas that do not give rise to renal injury.

The method comprises the steps of measuring the concentration of human NGAL in a sample of urine, preferably centrifuged to remove any neutrophils, from the individual to be diagnosed, and comparing the measured concentration with a selected cutoff value determined to exceed those urinary concentrations found in humans that have no renal disorder, but may either be apparently healthy or have other disorders including inflammatory conditions, bacterial infections or carcinomas. If the measured NGAL concentration exceeds the cutoff level, this is an indication that the human has suffered renal injury and may develop or has developed ARN, ATN or ATIN.

The cutoff level below which the urinary level of NGAL cannot be diagnostic of renal injury with an acceptable degree of specificity because such a level can be found in healthy individuals or those suffering from inflammatory, infective or cancerous conditions is preferably a level of 250 ng/mL or more, such a value between 250 ng/mL and 525 ng/mL, such as 275 ng/mL, or 300 ng/mL, or 325 ng/mL, or 350 ng/mL, or 375 ng/mL, or 400 ng/mL, or 425 ng/mL, or 450 ng/mL, or 475 ng/mL, or 500 ng/mL. In another embodiment, the cutoff value used is a value of 1 µg/mL or a higher value. Preferably, the positive predictive value for the urinary cutoff value is 80% or more, such as 85% or more, e.g. 90% or more. Alternatively, or in addition, the negative predictive value for the urinary cutoff is preferably 80% or more, such as 85% or more, e.g. 90% or more.

In another embodiment, the present invention comprises the steps of measuring the concentration of human NGAL in a sample of plasma or serum from the individual to be diagnosed, and comparing the measured concentration with a selected cutoff value determined to exceed those plasma or serum concentrations found in humans that have no renal disorder, but may either be apparently healthy or have other disorders including inflammatory conditions, bacterial infections or carcinomas. If the measured NGAL concentration exceeds the cutoff level, this is an indication that the human has suffered renal injury and may develop or has developed ARN, ATN or ATIN.

The cutoff level for the NGAL concentration in plasma or serum is similar to that for urine and is preferably a level of 250 ng/mL or more, e.g. 300 ng/mL or a higher value, or a value between 250 ng/mL and 525 ng/mL, such as 275 ng/mL, or 300 ng/mL, or 325 ng/mL, or 350 ng/mL, or 375 ng/mL, or 400 ng/mL, or 425 ng/mL, or 450 ng/mL, or 475 ng/mL, or 500 ng/mL. Preferably, the positive predictive value for the plasma cutoff value is 80% or more, such as 85% or more, e.g. 90% or more. Alternatively, or in addition, the negative predictive value for the plasma cutoff value chosen is preferably 80% or more, such as 85% or more, e.g. 90% or more.

A further aspect is that the method can be used to distinguish severe renal insults that are likely to require some form of dialysis, which typically give rise to very high urinary levels of NGAL, from less severe renal insults that may only occasionally require dialysis, which typically give rise to lower elevations of urinary NGAL.

Thus, a second, higher cutoff level, below which the urinary level of NGAL is not predictive of dialysis requirement but is diagnostic of a lesser degree of renal injury, is preferably a level between 1000 ng/mL and 3000 ng/mL, such as 1250 ng/mL, or 1500 ng/mL, or 1750 ng/mL, or 2000 ng/mL, or 2250 ng/mL, or 2500 ng/mL, or 2750 ng/mL. Preferably, the positive predictive value for the higher cutoff value is 80% or more, such as 85% or more, e.g. 90% or more. Alternatively, or in addition, the negative predictive value for the higher cutoff value is preferably 70% or more.

A further aspect is that the method can be used to detect the onset of renal affection in a patient who is under observation and/or treatment for another disease which may or may not itself be associated with an elevation in bodily fluid levels of NGAL, and in which renal affection is a possible complication. In this situation the urinary or plasma or serum concentrations of NGAL that are associated with the patient's underlying condition can be monitored daily or at shorter intervals, and the onset of renal affection will be indicated by a rise in the urinary or plasma or serum concentration of NGAL over the preceding levels. In these circumstances, the magnitude of the rise in NGAL concentrations indicative of the onset of renal affection is preferably 50 ng/mL or a more, such as 100 ng/mL or more, e.g. 150 ng/mL or more, such as 200 ng/mL or more, e.g. 300 ng/mL or more, such as 400 ng/mL or more, e.g. 500 ng/mL or more.

A further aspect is that the method can be used to monitor the course of renal affections giving rise to raised NGAL levels, both in their natural evolution and in response to therapeutic measures. In these circumstances, the change in NGAL levels will reflect the status of renal injury or regeneration, provided that any concurrent inflammatory, infectious or cancerous condition remains relatively stable during the period of monitoring. The intervals at which samples of bodily fluids are taken for monitoring can be short, thus providing the earliest possible indication of renal injury and thus permitting the early institution of therapeutic measures. Monitoring of NGAL levels in bodily fluids to detect renal affection is preferably carried out at intervals not longer than 24 hours, and more preferably at shorter intervals down to a suggested period of not longer than 3 h, or even shorter for instance if an insult is known to have occurred, e.g. during a surgical procedure.

Measurement of human NGAL in a sample of bodily fluid, such as a urine sample, can be performed by any method that provides satisfactory analytical specificity, sensitivity and precision. Preferred methods are binding assays using one or more binding molecules specific to human NGAL. Such binding molecules include, but are not limited to, polyclonal or monoclonal antibodies against NGAL or specific NGAL binding molecules generated by other means.

In a preferred method, monoclonal antibodies raised against recombinant human NGAL are used. One antibody is linked to a solid support to capture NGAL from a sample, such as a urine sample, while the other is linked to a label such as a dye complex, or biotin or enzyme that can be detected by any of many methods known to those skilled in the art. The solid support may e.g. be a polystyrene or polyvinyl chloride surface for enzyme-linked immunosorbent assay (ELISA), or latex (polystyrene) particles, or a filter frit composed of compressed polyethylene particles, or a porous nitrocellulose matrix, or indeed any suitable support used in immunochemical analyses.

A preferred means for measuring NGAL in accordance with the present invention in a sample of human urine comprises a dipstick, lateral flow or minicolumn test, which allows for the rapid, near-patient analysis of a sample. As will be understood by those of skill in the art upon reading this disclosure, however, other means for measuring NGAL can be used.

In a preferred embodiment, the method of the invention does not comprise a surgical, therapeutic or diagnostic step practiced on the human or animal body.

The following non-limiting examples are provided to further illustrate the present invention.

### EXAMPLES

### Example 1: NGAL Dipstick Test

The analytical area of a dipstick comprised of a polystyrene surface is coated with a capture antibody against human NGAL. An aliquot of the centrifuged, diluted sample is added to a solution of enzyme-labeled detection antibody against NGAL in the first tube, into which the dipstick is immersed. Complexes of enzyme-labeled detection antibody with NGAL are bound to the dipstick, which is then washed with tap water and placed in a chromogenic substrate solution in a second tube. The color developed in the substrate solution within a given time is read either by eye and compared with a chart of color intensities which indicates the concentration of NGAL in the urine sample, or in a simple colorimeter that can, for example, be programmed to indicate the NGAL concentration directly.

### Example 2: NGAL Lateral Flow Device

A lateral flow device comprised of a strip of porous nitrocellulose is coated near its distal end with a capture antibody against NGAL applied as a transverse band. A further transverse band of antibody against antibodies of the species from which the detection antibody is derived is placed distally to the capture antibody band and serves as a control of strip function. The proximal end of the strip contains the detection antibody against NGAL adsorbed or linked to labeled polystyrene particles or particles of dye complex. When an aliquot of the centrifuged urine sample is applied to the proximal end of the strip, the labeled particles attached to detection antibody travel along the strip by capillary attraction. When reaching the band of capture antibody, only those particles which have bound NGAL will be retained, giving rise to a detectable band. Particles reaching the control band of antibody against the detection antibody will produce a detectable band whether or not any NGAL has been bound. The intensity of the labeled bands can be read by eye in the case of colored particles or by means of the appropriate detection device for the label used. A positive result is indicated by color development or the accumulation of label in both bands, while a negative result is indicated by color development or other label only in the control band. Failure of color development or other label in the control band indicates inadequate strip function. The sensitivity of the test can be regulated by the dilution of the sample applied, which is adjusted so that only NGAL concentrations above the determined cutoff values give rise to a positive result. The sensitivity of the test can also be adjusted by linking the detection antibody to a mixture of labeled and unlabelled particles. Batches of strips can be pre-calibrated and equipped with a calibration code that can be read by the detection device, so that a quantitative or semiquantitative result can be read from the device. Many variations of the individual aspects of this lateral flow technology are possible, as known to those skilled in the art.

### Example 3: NGAL Minicolumn Test

A minicolumn contains a frit made of compressed polyethylene particles allowing the passage of fluid and cells. The frit is coated with capture antibody against human NGAL. The minicolumn is incorporated into a device, which by means of automated liquid handling allows the diluted sample to be applied at a fixed flow rate and volume, followed by detection antibody complexed with dye. After the passage of wash solution, the color intensity of the frit is read by light diffusion photometry. The batches of frits are pre-calibrated and the minicolumns equipped with a calibration code that can be read by the device, so that a quantitative result can be displayed by the instrument without the need for prior calibration with standards.

### Example 4: NGAL Sandwich ELISA

Purified recombinant human NGAL for use as a standard and as calibrator material was prepared as described by Kjeldsen et al. (1996). Antibodies against NGAL were those described by Kjeldsen et al. (1993; 1996). Polystyrene ELISA plates were coated overnight at 4°C with antibody 211-1 at a concentration of 2 µg/mL in 0.05 M sodium carbonate buffer, pH 9.4, applied at 100 µL/well. The wells were emptied, washed 3 times with wash buffer of phosphate-buffered saline, pH 7.4, containing 0.05% Tween 20, and blotted. Dilutions of calibrator and samples in dilution buffer (wash buffer containing bovine albumin at 0.1 mg/mL) were applied to the wells in 100-µL volumes and incubated for 1 hour at room temperature on a shaking table. The wells were then emptied, washed and blotted as before. Biotinylated antibody 211-2 at 0.25 µg/mL in dilution buffer was added to each well at 100 µL/well and incubated for 1 hour at room temperature on a shaking table. The wells were then emptied, washed and blotted as before. A complex of horseradish peroxidase and streptavidin (Zymed, CA) at a dilution of 1/2000 in dilution buffer was added to each well at 100 µL/well and incubated for 1 hour at room temperature on a shaking table. The wells were then emptied, washed and blotted as before. A substrate solution containing tetramethylbenzidine and peroxide (TMB-ONE, Kem-En-Tech, Denmark) was then applied to each well at 100 µL/well and incubated at room temperature in the dark for exactly 8 minutes, after which the color reaction was stopped by adding 50 µL of 1 M sulfuric acid to each well. The light absorbances of the wells at a wavelength of 450 nm were then read in an ELISA plate reader, subtracting the light absorbances at 650 nm. The concentrations of NGAL in the samples were then calculated from the standard curve generated from the light absorbance readings of the calibrators of known concentration.

The assay had a range of 0.02 ng/mL to 1 ng/mL, with a detection limit (95% confidence limit of difference from zero) of 2.4 pg/mL, and showed parallelism between dilutions of purified calibrator and samples. The concentration of NGAL was 90 ng/mL in a pool of normal human serum and 5.4 ng/mL in a pool of normal human urine.

Further examples show clinical and paraclinical correlations, together with an estimate of the diagnostic value with respect to renal disorders, of NGAL determination in serum or plasma and in urine from unselected adult patients admitted to a hospital intensive care unit. NGAL was determined by a sandwich ELISA similar in all essential details to that described in Example 4 above.

### Example 5: Initial clinical and paraclinical correlations

Concentrations of NGAL in single spot samples of urine and serum from 11 unselected adult patients admitted to a hospital intensive care unit are shown in Table 1, where they are compared with clinical and paraclinical data.

**Table 1. NGAL concentrations in urine and serum from 11 patients: correlations with clinical and paraclinical data**

| **Pt. no.** | **Diagnoses** | **u-NGAL rank order ng/mL** | **s-NGAL rank order ng/mL** | **p-creatinine rank order µM** | **Neutroph. rank order x 10⁹/mL** | **s-CRP rank order µg/mL** |
|---|---|---|---|---|---|---|
| 1 | MT, oliguria | 10 | 10 | 10 | 11 | 2 |
| | | 8640 | 435 | 366 | 15.6 | 31 |
| 2 | Rupt. aortic an., anuria, hemodialysis | 7 | 9 | 11 | 7 | 6 |
| | | 1490 | 342 | 605 | 9.6 | 71 |
| 3 | B-cell ALL, pneumonia | 9 | 8 | 2 | 2 | 10 |
| | | 3800 | 330 | 63 | 2.0 | 97 |
| 4 | Fecal peritonitis | 8 | 7 | 7 | 9 | 9 |
| | | 2700 | 273 | 108 | 16 | 87 |
| 5 | *S. aureus* sepsis, ATN | 11 | 11 | 6 | 8 | 8 |
| | | 15,700 | 922 | 103 | 13.9 | 81 |
| 6 | AML, septic shock | 6 | 5 | 9 | 1 | 4 |
| | | 1030 | 146 | 164 | <0.1 | 56 |
| 7 | HRS *E. faecalis* bacteremia | 3 | 3 | 3 | 5 | 1 |
| | | 900 | 92 | 82 | 6.7 | 19 |
| 8 | Hematoma, ARDS | 5 | 4 | 8 | 6 | 3 |
| | | 1000 | 114 | 154 | 9.5 | 37 |
| 9 | MT | 1 | 1 | 4 | 4 | 11 |
| | | 110 | 66 | 91 | 5.7 | 103 |
| 10 | MT | 4 | 6 | 5 | 3 | 7 |
| | | 950 | 147 | 98 | 5.0 | 81 |
| 11 | Splenectomy, perforated diaphragm | 2 | 2 | 1 | 10 | 5 |
| | | 130 | 79 | 30 | 19 | 62 |

| | | | | | | |
|---|---|---|---|---|---|---|
| Normal values: s-NGAL 90 ng/mL, u-NGAL 5.4 ng/mL ALL: acute lymphatic leukemia; AML: acute myeloid leukemia; ARDS: adult respiratory distress syndrome; ATN: acute tubular necrosis; CRP: C-reactive protein; HRS: hepato-renal syndrome; MT: multiple trauma; p: plasma; s: serum; u: urine | | | | | | |

**Spearman's coefficients of rank correlation (r)**
u-NGAL/s-NGAL: r 0.945 *P* <<0.001
u-NGAL/p-creatinine: r 0.418 *P* 0.1
s-NGAL/neutrophil count: r 0.273, not significant
s-NGAL/s-CRP: r 0.064, not significant

All the patients had raised urinary concentrations of NGAL. There was a very close correlation between serum and urinary NGAL levels, but only a moderate correlation between urinary NGAL and plasma creatinine. The case that was clinically diagnosed as ATN was characterized by an extremely high urinary NGAL at a time when there had been no significant rise in plasma creatinine. The poor correlation between serum NGAL and the neutrophil count shows that serum NGAL is not a reflection of neutrophilia. In particular, both the urine and serum NGAL were raised in patient no. 3, with a neutrophil count of only 2.0 x 10⁹/mL because of acute lymphatic leukemia, and in patient no. 6, with an uncountably low number of neutrophils because of acute myeloid leukemia. Furthermore, the lack of correlation with CRP shows that s-NGAL is not just an acute phase protein. Repeated analyses of stored samples showed that NGAL may have limited stability in urine samples, while being more stable in serum samples. The results suggest that NGAL is produced in organ damage, particularly, but not only, by the kidneys. It spills over into the blood and is excreted in the urine. However, renal NGAL in ATN also passes directly into the urine to produce a very high urinary concentration of NGAL.

Patient nos. 3 and 4, who at the time of sampling had no clinical diagnosis of renal disorder and whose p-creatinine was at that time within the normal range, had levels of urinary NGAL that were higher than that of patient no. 2, who was receiving hemodialysis for anuria after rupture of an aortic aneurysm. The two patients (nos. 3 and 4) may have developed renal ischemic injury because of their severe infections, the rise in urinary NGAL preceding any rise in p-creatinine, as was also observed in patient no. 5, who was clinically diagnosed as having ATN.

### Example 6: Diagnostic power with respect to renal disorder of urine and plasma NGAL determinations in unselected adult patients admitted to intensive care

NGAL was determined in urine and plasma samples collected each morning from 109 consecutive patients admitted to a hospital intensive care unit. On the basis of discharge summaries and the results of routine blood tests, it was possible to classify (blindly with respect to NGAL data) 60 of these patients into those with and without a renal affection during their admission. Incomplete data made it impossible to classify the remaining 49 patients with sufficient certainty, and these patients were excluded from the analysis. Maximal NGAL concentrations in urine and plasma from the 60 clinically classifiable patients are given in Table 2. The diagnostic power with respect to renal affection of the maximal concentration of NGAL reached in urine and plasma from each classified patient was then determined by plotting receiver operating characteristic (ROC) curves for the urine and plasma values. The time courses of the daily urine and plasma NGAL values were also plotted for each patient and compared with paraclinical data such as the plasma creatinine values.

**Table 2. Maximal NGAL concentrations in plasma and urine from 60 patients admitted to intensive care that were clinically classifiable as with or without renal affection**

| **Patient number** | **plasma NGAL ng/mL** | **urine NGAL ng/mL** | **Clinical classification** | | | |
|---|---|---|---|---|---|---|
| | | | **Renal affection** | **Sepsis** | **Cancer** | **Hemodialysis** |
| 1 | 92 | 50 | - | - | - | - |
| 2 | 1005 | 5000 | + | - | - | + |
| 3 | 151 | 183 | - | + | - | - |
| 6 | 1320 | 5000 | + | + | - | + |
| 7 | 777 | 3229 | + | + | + | + |
| 8 | 712 | 269 | + | + | + | - |
| 9 | 2941 | 5000 | + | - | - | + |
| 10 | 3092 | 5000 | + | - | - | + |
| 11 | 65 | 13 | - | - | - | - |
| 12 | 2117 | 5000 | + | + | - | + |
| 15 | 546 | 3128 | + | + | + | - |
| 17 | 110 | 10 | + | - | - | - |
| 18 | 135 | 68 | - | - | - | - |
| 19 | 191 | 2672 | + | + | + | + |
| 20 | 336 | 304 | - | - | + | - |
| 21 | 1434 | 5000 | + | + | - | + |
| 24 | 307 | 1042 | + | - | - | + |
| 29 | 71 | 50 | - | - | - | - |
| 30 | 320 | 874 | - | - | - | - |
| 39 | 1416 | 1073 | + | + | - | - |
| 41 | 181 | 42 | - | - | - | - |
| 42 | 115 | 29 | - | - | - | - |
| 43 | 436 | 176 | + | - | - | - |
| 44 | 446 | 680 | + | - | - | - |
| 45 | 270 | 37 | - | - | - | - |
| 46 | 1962 | 3222 | + | + | + | + |
| 47 | 222 | 89 | - | + | - | - |
| 48 | 1040 | 3974 | + | - | - | + |
| 49 | 228 | 48 | | - | - | - |
| 51 | 256 | 519 | + | - | + | + |
| 52 | 294 | 685 | + | + | + | - |
| 53 | 586 | 1337 | + | + | + | - |
| 54 | 1376 | 2915 | + | - | + | + |
| 55 | 1276 | 5000 | + | + | + | + |
| 57 | 180 | 24 | - | - | - | - |
| 58 | 716 | 3431 | + | - | - | + |
| 59 | 108 | 9 | - | + | - | - |
| 60 | 1219 | 2713 | + | + | + | + |
| 65 | 460 | 1705 | - | - | + | - |
| 67 | 1470 | 5000 | + | - | - | + |
| 69 | 318 | 68 | - | + | - | - |
| 72 | 175 | 17 | - | - | - | - |
| 75 | 645 | 3360 | + | - | + | + |
| 80 | 322 | 328 | - | + | + | - |
| 82 | 216 | 30 | - | + | - | - |
| 83 | 259 | 34 | - | + | - | - |
| 84 | 25 | 20 | - | - | - | - |
| 85 | 1067 | 370 | + | + | - | + |
| 86 | 64 | 21 | - | - | - | - |
| 87 | 276 | 779 | + | - | - | - |
| 88 | 302 | 1024 | - | + | - | - |
| 91 | 236 | 17 | - | - | - | - |
| 93 | 1595 | 5000 | + | + | - | + |
| 97 | 3491 | 708 | + | + | + | + |
| 99 | 820 | 401 | + | + | - | + |
| 100 | 354 | 85 | - | - | + | - |
| 101 | 1144 | 5000 | + | + | + | + |
| 104 | 1844 | 2748 | + | + | - | + |
| 105 | 488 | 4473 | + | - | - | + |
| 108 | 111 | 46 | - | + | - | - |

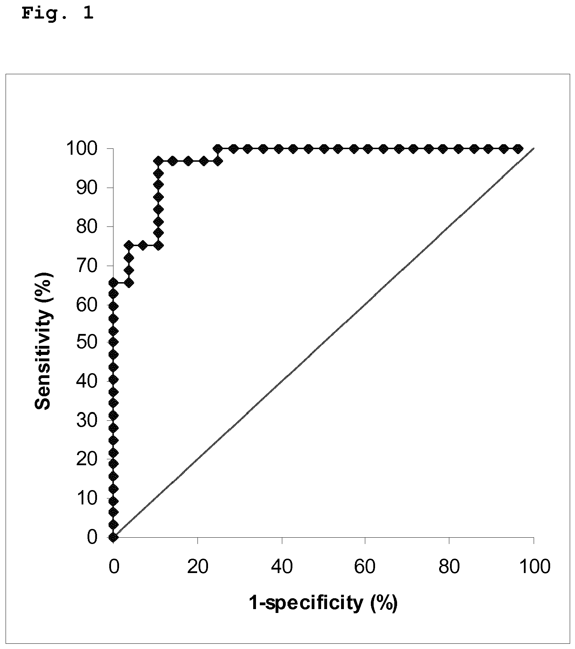

Figure 1 shows the ROC curve for maximal urinary NGAL values with respect to the diagnosis of renal affection. The area under the curve was 0.930 and the cutoff value below which the concentration of urinary NGAL is not diagnostic of renal disorder was determined to be between 370 ng/mL and 329 ng/mL. With a cutoff in this range the diagnostic sensitivity was 96.9%, the diagnostic specificity was 89.3%, the positive predictive value was 91.2% and the negative predictive value was 96.2%.

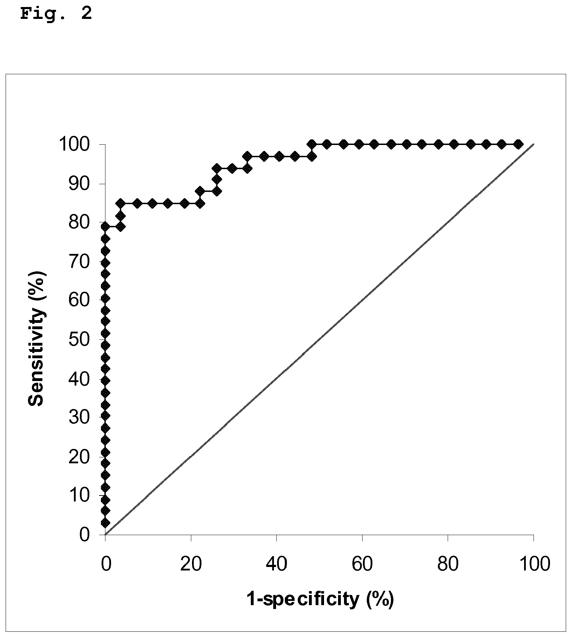

Figure 2 shows the ROC curve for maximal plasma NGAL values with respect to the diagnosis of renal affection. The area under the curve was 0.914 and the cutoff value below which the concentration of plasma NGAL is not diagnostic of renal disorder was determined to be between 436 ng/mL and 355 ng/mL. With a cutoff in this range the diagnostic sensitivity was 84.8%, the diagnostic specificity was 96.3%, the positive predictive value was 93.1% and the negative predictive value was 83.9%.

### Diagnosis of dialysis requirement

Patients with renal affection fell into two groups with respect to the maximal urinary NGAL values observed. The first group (11 patients) was characterized by urinary NGAL values of 1337 ng/mL or less, and contained patients with lesser degrees of renal affection as judged by other clinical and paraclinical data. In this group 3 patients received some form of hemodialysis. The second group (21 patients) was characterized by urinary NGAL values of 2672 ng/mL or more and contained 17 patients clinically diagnosed as having ATN or ATIN. In this group 20 patients required some form of hemodialysis. The cutoff value below which the urinary NGAL concentration is not predictive of dialysis need but may be diagnostic of a lesser degree of renal disorder is therefore between 1338 and 2672 ng/mL.

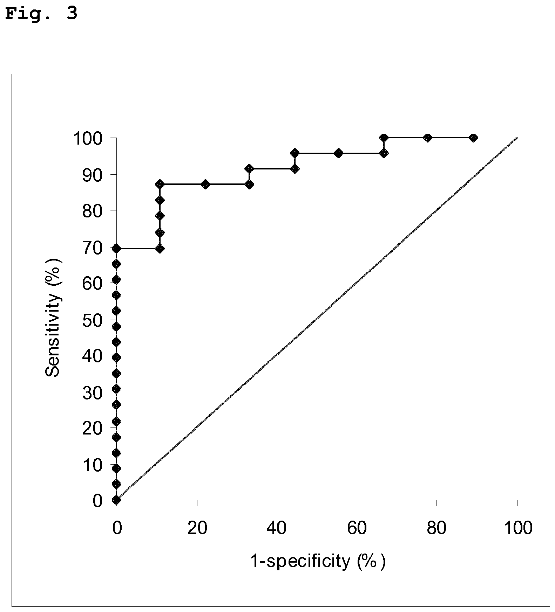

Figure 3 shows the ROC curve for maximal urinary NGAL values with respect to the diagnosis of renal affection requiring dialysis. The area under the curve was 0.807 and the cutoff value below which the concentration of urinary NGAL is not diagnostic of dialysis requirement was confirmed to be between 1338 ng/mL and 2672 ng/mL. With a cutoff in this range the diagnostic sensitivity was 87.0%, the diagnostic specificity was 88.9%, the positive predictive value was 95.2% and the negative predictive value was 72.7%.

### Time course of NGAL values

When the time course of daily urinary and plasma NGAL values was plotted and compared with the clinical and paraclinical data in individual classified patients, it was observed that rises of 86 ng/mL, 125 ng/mL and 200 ng/mL in urine NGAL over the preceding level were associated with a deterioration of renal function as recorded in the discharge summary or shown by a rising plasma creatinine level. Rises in urinary NGAL of more than 200 ng/mL above the preceding level were also associated with decline in renal function when these rises brought the urinary NGAL level above the cutoff level of 329 ng/mL. The number of patients clearly showing this type of intercurrent rise in urinary NGAL was not large enough to permit a meaningful analysis of the diagnostic value with respect to acute renal failure in relation to the magnitude of the rise.

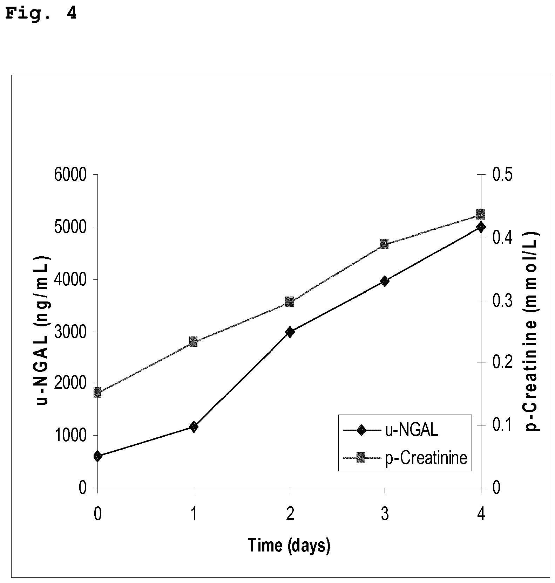

In some cases there was a parallelism between the rise in urinary NGAL and the rise in plasma creatinine attributed to declining renal function. Figure 4 shows such a parallel rise in urinary NGAL and plasma creatinine in an elderly male patient operated acutely for rupture of an abdominal aortic aneurysm. Here the ischemic insult to the kidneys due to hemorrhage and aortic clamping took place on the day preceding the first plasma and urine samples, so that the initial levels of urinary NGAL and plasma creatinine were both indicative of a degree of renal failure.

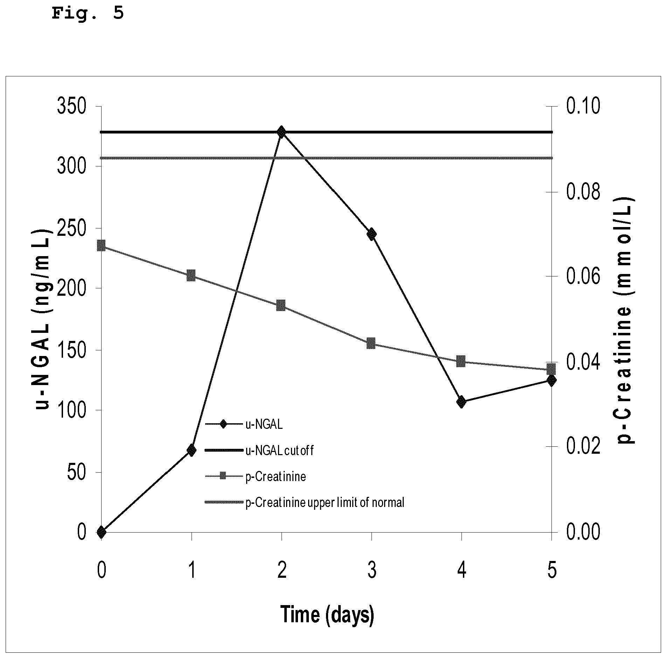

A rise in urinary NGAL of 200 ng/mL or more over the preceding value, but to a value that remains below the cutoff level of 329 ng/mL, is not diagnostic of acute renal failure, as it may be due to another condition, such as sepsis, which does not necessarily affect the kidneys. Figure 5 shows such a rise in urinary NGAL which was associated with the development of a sepsis that did not affect renal function, as supported by the fact that the plasma creatinine level remained well within the normal range throughout the clinical course in the intensive care unit.

### References

Aulitzky WK, Schlegel PN, Wu DF, Cheng CY, Chen CL, Li PS, Goldstein M, Reidenberg M, Bardin CW (1992) Measurement of urinary clusterin as an index of nephrotoxicity. Proc Soc Exp Biol Med 199:93-96.
Han WK, Bailly V, Abichandani R, Thadhani R, Bonventre JV (2002) Kidney Injury Molecule-1 (KIM-1): a novel biomarker for human renal proximal tubule injury. Kidney Int 62:237-244.
Kjeldsen L, Johnsen AH, Sengelov H, Borregaard N (1993) Isolation and primary structure of NGAL, a novel protein associated with human neutrophil gelatinase. J Biol Chem 268:10425-10432.
Kjeldsen L, Koch C, Arnljots K, Borregaard N (1996) Characterization of two ELISAs for NGAL, a newly described lipocalin in human neutrophils. J Immunol Methods 198:155-164.
Kotanko P, Margreiter R, Pfaller W (2000) Urinary N-acetyl-beta-D-glucosaminidase and neopterin aid in the diagnosis of rejection and acute tubular necrosis in initially nonfunctioning kidney grafts. Nephron 84:228-235.
Liu Q, Nilsen-Hamilton M (1995) Identification of a new acute phase protein. J Biol Chem 270:22565-22570.
Monier F, Surla A, Guillot M, Morel F (2000) Gelatinase isoforms in urine from bladder cancer patients. Clin Chim Acta 299:11-23.
Muramatsu Y, Tsujie M, Kohda Y, Pham B, Perantoni AO, Zhao H, Jo SK, Yuen PS, Craig L, Hu X, Star RA (2002) Early detection of cysteine rich protein 61 (CYR61, CCN1) in urine following renal ischemic reperfusion injury. Kidney Int 62:1601-1610.
Penders J, Delanghe JR (2004) Alpha 1-microglobulin: clinical laboratory aspects and applications. Clin Chim Acta 346:107-118.
Stoesz SP, Gould MN (1995) Overexpression of neu-related lipocalin (NRL) in neu-initiated but not ras or chemically initiated rat mammary carcinomas. Oncogene 11:2233-2241.
Triebel S, Blaser J, Reinke H, Tschesche H (1992) A 25 kDa alpha 2-microglobulin-related protein is a component of the 125 kDa form of human gelatinase. FEBS Lett 314:386-388.
Tsuchida T, Eguchi N, Eguchi Y, Numabe A, Nakajima H, Oda H, Seiki K, Hakamada-Taguchi R, Urade Y, Uehara Y (2004) Lipocalin-type prostaglandin D synthase in urine in adriamycin-induced nephropathy of mice. Nephron Physiol 96:42-51.
Yan L, Borregaard N, Kjeldsen L, Moses MA (2001) The high molecular weight urinary matrix metalloproteinase (MMP) activity is a complex of gelatinase B/MMP-9 and neutrophil gelatinase-associated lipocalin (NGAL). Modulation of MMP-9 activity by NGAL. J Biol Chem 276:37258-37265.

## Claims

1. A method of discriminating between a condition that does not affect the kidney, wherein the condition that does not affect the kidney is an inflammatory disorder, an infective disorder or a cancerous disorder, and the likelihood of a renal disorder, wherein the renal disorder is selected from acute renal failure, acute tubular necrosis and acute tubule-interstitial nephropathy, in a human being, said method comprising the steps of:
i) determining the concentration of human neutrophil gelatinase-associated lipocalin (NGAL) in a sample of bodily fluid from the human being,
ii) comparing said concentration with a predetermined cutoff value, said cutoff value being chosen to exclude lower concentrations of NGAL associated with conditions that do not affect the kidney, wherein a concentration above the cutoff value is indicative of the likelihood of the renal disorder.

2. The method according to claim 1, wherein the bodily fluid is urine, plasma or serum.

3. The method according to any of the preceding claims, comprising the further step of repeating steps i) and ii) one or more times.

4. The method according to any of the preceding claims, comprising the further step of repeating steps i) and ii) within 24 hours, e.g. within 12 hours, such as within 6 hours, e.g. within 3 hours.

5. The method according to any of the preceding claims, comprising the further step of repeating steps i) and ii) after a treatment of the renal disorder has been initiated or completed.

6. The method according to any of the preceding claims, wherein the renal disorder is a post-ischemic renal injury.

7. The method according to any of the preceding claims, wherein the renal disorder is caused by a nephrotoxic agent.

8. The method according to any of the preceding claims, wherein NGAL is measured by means of a molecule that binds specifically to NGAL.

## Patentansprüche

1. Verfahren zum Unterscheiden zwischen einer Krankheit, die die Niere nicht beeinträchtigt, wobei es sich bei der Krankheit, die die Niere nicht beeinträchtigt, um eine entzündliche Erkrankung, eine infektiöse Erkrankung oder eine Krebserkrankung handelt, und der Wahrscheinlichkeit einer Nierenerkrankung, wobei die Nierenerkrankung aus akutem Nierenversagen, akuter tubulärer Nekrose und akuter tubulointerstitieller Nephropathie ausgewählt ist, bei einem Menschen, wobei das Verfahren die folgenden Schritte umfasst:
i) Bestimmen der Konzentration von humanem Neutrophil-Gelatinase-assoziiertem Lipocalin (NGAL) in einer Probe von Körperflüssigkeit von dem Menschen,
ii) Vergleichen der Konzentration mit einem vorbestimmten Cut-off-Wert, wobei der Cut-off-Wert ausgewählt ist, um niedrigere Konzentrationen von NGAL, die mit den Krankheiten assoziiert sind, die die Niere nicht beeinträchtigen, auszuschließen, wobei eine Konzentration oberhalb des Cut-off-Werts die Wahrscheinlichkeit der Nierenerkrankung angibt.

2. Verfahren nach Anspruch 1, wobei es sich bei der Körperflüssigkeit um Urin, Plasma oder Serum handelt.

3. Verfahren nach einem der vorhergehenden Ansprüche, das den weiteren Schritt eines ein- oder mehrmaligen Wiederholens der Schritte i) und ii) umfasst.

4. Verfahren nach einem der vorhergehenden Ansprüche, umfassend den weiteren Schritt eines Wiederholens der Schritte i) and ii) innerhalb von 24 Stunden, z. B innerhalb von 12 Stunden, wie etwa innerhalb von 6 Stunden, z. B. innerhalb von 3 Stunden.

5. Verfahren nach einem der vorhergehenden Ansprüche, umfassend den weiteren Schritt eines Wiederholens der Schritte i) und ii), nachdem eine Behandlung der Nierenerkrankung initiiert oder abgeschlossen worden ist.

6. Verfahren nach einem der vorhergehenden Ansprüche, wobei es sich bei der Nierenerkrankung um eine post-ischämische Nierenschädigung handelt.

7. Verfahren nach einem der vorhergehenden Ansprüche, wobei die Nierenerkrankung durch einen nephrotoxischen Wirkstoff verursacht ist.

8. Verfahren nach einem der vorhergehenden Ansprüche, wobei NGAL durch ein Molekül gemessen wird, das spezifisch an NGAL bindet.

## Revendications

1. Procédé de discrimination entre une pathologie qui n'affecte pas le rein, dans lequel la pathologie qui n'affecte pas le rein est un trouble inflammatoire, un trouble infectieux ou un trouble cancéreux, et la probabilité d'un trouble rénal, dans lequel le trouble rénal est sélectionné parmi une insuffisance rénale aiguë, une nécrose tubulaire aiguë et une néphropathie tubulo-interstitielle aiguë, chez un être humain, ledit procédé comprenant les étapes :
i) de détermination de la concentration de la lipocaline neutrophile humaine associée à la gélatinase (NGAL) dans un échantillon de fluide corporel de l'être humain,
ii) de comparaison de ladite concentration avec une valeur seuil prédéterminée, ladite valeur seuil étant choisie pour exclure des concentrations de NGAL inférieures associées à des pathologies qui n'affectent pas le rein, dans lequel une concentration au-dessus de la valeur seuil indique la probabilité du trouble rénal.

2. Procédé selon la revendication 1, dans lequel le fluide corporel est l'urine, le plasma ou le sérum.

3. Procédé selon l'une quelconque des revendications précédentes, comprenant l'étape supplémentaire de répétition des étapes i) et ii) une ou plusieurs fois.

4. Procédé selon l'une quelconque des revendications précédentes, comprenant l'étape supplémentaire de répétition des étapes i) et ii) dans les 24 heures, par exemple dans les 12 heures, tel que dans les 6 heures, par exemple dans les 3 heures.

5. Procédé selon l'une quelconque des revendications précédentes, comprenant l'étape supplémentaire de répétition des étapes i) et ii) après qu'un traitement du trouble rénal a été initié ou achevé.

6. Procédé selon l'une quelconque des revendications précédentes, dans lequel le trouble rénal est une lésion rénale post-ischémique.

7. Procédé selon l'une quelconque des revendications précédentes, dans lequel le trouble rénal est causé par un agent néphrotoxique.

8. Procédé selon l'une quelconque des revendications précédentes, dans lequel la NGAL est mesurée au moyen d'une molécule qui se lie spécifiquement à la NGAL.
